# EUROPEAN PATENT SPECIFICATION

(11) **EP 4 449 344 B1**
(45) Date of publication and mention of the grant of the patent: **01.04.2026**
(21) Application number: 22830788.0
(22) Date of filing: 07.12.2022
(51) Int. Cl.: G06T 7/00, G06T 11/00

(54) **GENERATION OF ADDITIONAL VIEWS IN BODY PART X-RAY IMAGING**
ERZEUGUNG ZUSÄTZLICHER ANSICHTEN IN DER KÖRPERTEILRÖNTGENBILDGEBUNG
GÉNÉRATION DE VUES SUPPLÉMENTAIRES DANS UNE IMAGERIE PAR RAYONS X DE PARTIES CORPORELLES

(30) Priority: 16.12.2021 RU 2021137338
(43) Date of publication of application: 23.10.2024
(73) Proprietor: Koninklijke Philips N.V., 5656 AG Eindhoven (NL)
(72) Inventor: SAALBACH, Axel, 5656 AG Eindhoven (NL); SCHULZ, Heinrich, 5656 AG Eindhoven (NL); SIRAZITDINOV, Ilyas, 5656 AG Eindhoven (NL)
(74) Representative: Philips Intellectual Property & Standards
(86) International application number: PCT/EP2022/084687
(87) International publication number: WO 2023/110553

(56) References cited:
- SHEN L ET AL: "Novel-View X-Ray Projection Synthesis Through Geometry-Integrated Deep Learning", INTERNATIONAL JOURNAL OF RADIATION: ONCOLOGY BIOLOGY PHYSICS, PERGAMON PRESS, USA, vol. 111, no. 3, 22 October 2021 (2021-10-22), XP086838422, ISSN: 0360-3016, DOI: 10.1016/J.IJROBP.2021.07.534
- CHENG PENG ET AL: "XraySyn: Realistic View Synthesis From a Single Radiograph Through CT Priors", ARXIV.ORG, CORNELL UNIVERSITY LIBRARY, 201 OLIN LIBRARY CORNELL UNIVERSITY ITHACA, NY 14853, 4 December 2020 (2020-12-04), XP081829942
- RODIN IVAN ET AL: "Multitask and Multimodal Neural Network Model for Interpretable Analysis of X-ray Images", 2019 IEEE INTERNATIONAL CONFERENCE ON BIOINFORMATICS AND BIOMEDICINE (BIBM), IEEE, 18 November 2019 (2019-11-18), pages 1601 - 1604, XP033704102, DOI: 10.1109/BIBM47256.2019.8983272
- HADRIEN BERTRAND ET AL: "Do Lateral Views Help Automated Chest X-ray Predictions?", ARXIV.ORG, CORNELL UNIVERSITY LIBRARY, 201 OLIN LIBRARY CORNELL UNIVERSITY ITHACA, NY 14853, 18 April 2019 (2019-04-18), XP081170870

## Description

### FIELD OF THE INVENTION

The present invention relates to medical imaging, and in particular to an image processing apparatus, to an imaging processing method, to a computer program element, and to a computer-readable data carrier.

### BACKGROUND OF THE INVENTION

X-rays have been used for medical imaging. Projection radiographs are useful in the detection of pathology of the skeletal system as well as for detecting some disease processes in soft tissue. Different views (also known as projections) of the body part can be obtained by changing the relative orientation of the body and the direction of the X-ray beam.

For example, chest X-ray is the most employed type of medical imaging study. During the examination, different views may be acquired to facilitate the diagnosis. Depending on the indication and clinical guidelines this can include posterior-anterior (PA) or anterior-posterior (AP), lateral (LAT) and other views. However, while several studies, e.g., Hadrien et al. (2019) "Do Lateral Views Help Automated Chest X-ray Predictions?", suggest the value of LAT images, they are not always available when required. Therefore, the initial indication may suggest only the acquisition of a PA image for various reasons, e.g., due to dose concerns, or because of the presumed condition of the patient. Another reason could be that LAT images might also be difficult to obtain (e.g., in bedside imaging).

Therefore, in some cases, a new examination would have to be schedule for the patient for acquiring an additional view of the body part for diagnostic purposes later in the workflow. This may cause substantial organizational overhead, and negative patient experience.

Alternatively, Shen et al. (2021) "Novel-View X-Ray Projection Synthesis Through Geometry-Integrated Deep Learning" discloses obtaining an X-ray projection image at a novel view angle from a given projection image at a specific view angle to alleviate the need for actual projection measurement and Peng et al. (2020) "XraySyn: Realistic View Synthesis From a Single Radiograph Through CT Priors" discloses a method to synthesize novel views on real radiographs.

Rodin et al. (2019) "Multitask and Multimodal Neural Network Model for Interpretable Analysis of X-ray Images" discloses a model that generates a short textual summary with essential information on the found pathologies along with their location and severity

### SUMMARY OF THE INVENTION

There may, therefore, be a need to improve X-ray imaging workflow.

The invention is defined by the independent claims. The dependent claims define advantageous embodiments.

According to a first aspect of the present invention, there is provided an image processing apparatus according to claim 1.

The machine learning-based approach as described herein can generate additional synthetic view(s) from an acquired image. For example, a synthetic lateral view of the chest may be generated from an acquired X-ray image having the PA view. While such an approach might not be able to predict pathologies obscured in the original view, the synthetic additional view(s) may provide the most likely representation based on large amounts of clinical data. Thus, the synthetic X-ray image having a different view of the body part may allow a radiologist to further assess pathologies that may be present in the acquired image, without performing a new examination for the patient. This could reduce the dose without (or with less) compromising diagnosis and therapy.

The first X-ray image is an X-ray image acquired in an image acquisition. The first X-ray image may also be referred to as real X-ray image or acquired X-ray image.

The second X-ray image is an X-ray image generated from the first X-ray image. The second X-ray image may also be referred to as synthetic X-ray image or generated X-ray image.

Examples of the body part may include, but are not limited to, joints, neck, spine, chest, limbs, and other parts of the body.

The "machine learning model", as used herein, may refer to a statistical method that enables machines to "learn" tasks from data without explicitly programming, relying on patterns in the data instead. For example, the machine learning model may be a deep learning model. Deep learning is a subset of machine learning modeled loosely on the neural pathways of the human brain. Deep refers to the multiple layers between the input and output layers. In deep learning, the algorithm automatically learns what features are useful. A general introduction into machine learning and corresponding software frameworks is described in "Machine Learning and Deep Learning frameworks and libraries for large-scale data mining: a survey"; Artificial Intelligence Review; Giang Nguyen et al., June 2019, Volume 52, Issue 1, pp 77-124. The machine-learning model may be a machine learning generative model for reproducing a mapping between the first X-ray image having a first view of the body part and the second X-ray image having a second view of the body part. The machine learning generative model may be used to e.g. generation additional views in form of synthetic data from the first X-ray image (e.g. PA data).

According to claim 1, the second X-ray image is generated based on the probability of the presence of one or more suspicious pathologies in the first X-ray image. This may allow a radiologist to further assess the suspicious pathologies in a different view.

According to an embodiment of the present invention, the input is further configured to receive non-image patient data of the patient. The pre-trained machine-learning model is further configured to apply (use) the received non-image patient data to generate the second X-ray image.

In other words, in addition to the first X-ray image, non-image patient data may be included into the training and inference process to improve the prediction.

Examples of the non-image patient data may include, but are not limited to, age of the patient, gender of the patient, presence of implants, medication record associated with the patient, or any combination thereof.

According to an embodiment of the present invention, the input is further configured to receive system data of an X-ray imaging apparatus for acquiring the first X-ray image of the patient. The pre-trained machine-learning model is further configured to apply (use) the received system data to generate the second X-ray image.

In addition to the first X-ray image data, system data may also be included into the training and generation process to improve the prediction.

The system data may comprise e.g., a view position, one or more acquisition parameters (such as kVp), and any other system data.

According to an embodiment of the present invention, the pre-trained machine-learning model comprises an encoder-decoder architecture.

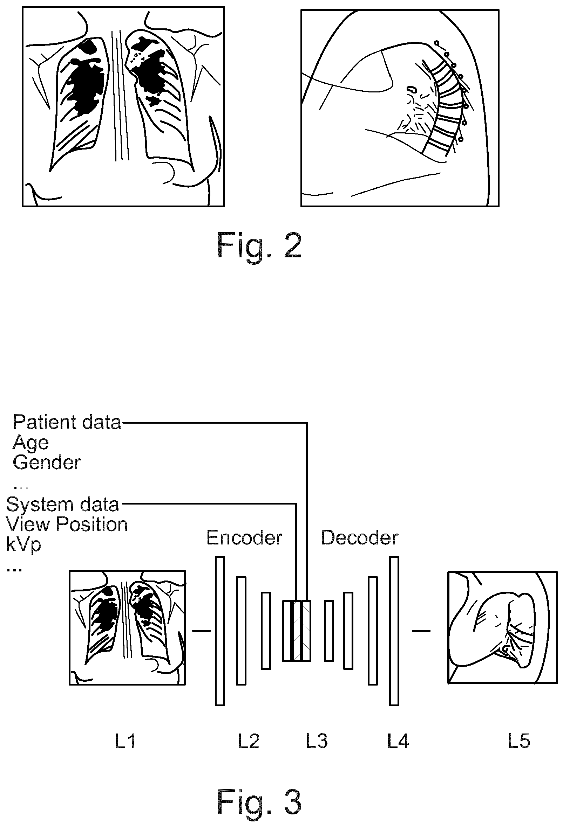

An exemplary encoder-decoder architecture is illustrated in Fig. 3.

According to an embodiment of the present invention, the pre-trained machine-learning model comprises a generator component and a discriminator component. The generator component comprises the encoder-decoder architecture configured to map the first X-ray image to the second X-ray image. The discriminator component comprises a discriminator that has been trained with concatenated image pairs to discriminate both, wherein each concatenated image pair comprises a first X-ray image pair comprising first and second X-ray images acquired by an X-ray imaging device, and a second X-ray image pair comprising the first X-ray image acquired by the X-ray imaging device and a second X-ray image generated by the image processing apparatus.

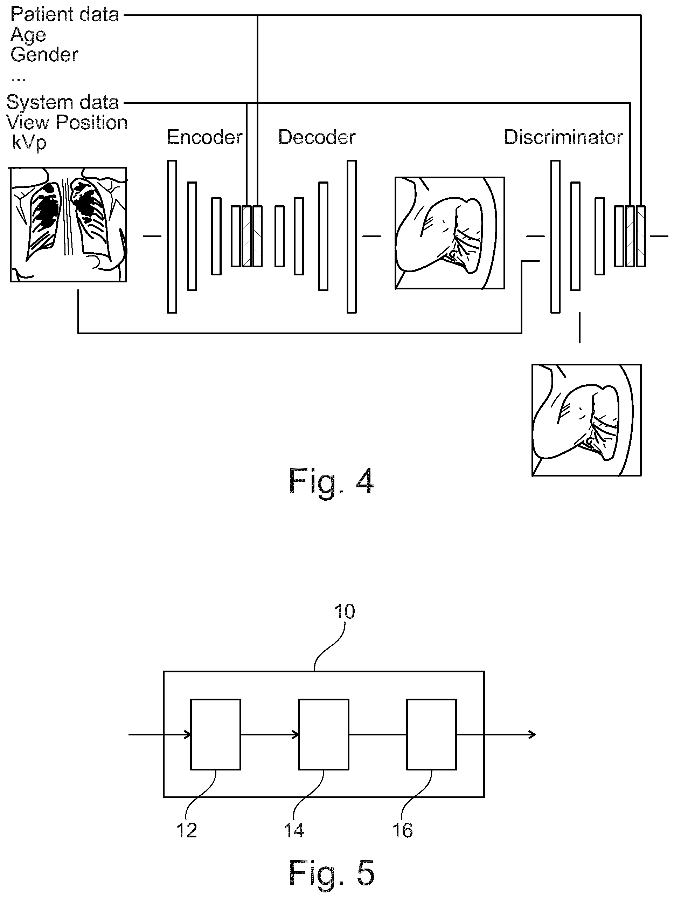

This will be explained in detail hereinafter and particularly with respect to the example shown in Fig. 4.

According to an embodiment of the present invention, the processor is further configured to detect a presence of one or more pathologies in the second X-ray image.

According to an embodiment of the present invention, the processor is further configured to provide a probability score of the one or more detected pathologies in the second X-ray image.

Accordingly, the detection of pathologies in the additional view may facilitate the diagnosis process. For example, LAT images may be beneficial for pneumonia detection and other pathologies.

According to a second aspect of the present invention, there is provided an X-ray imaging system. The X-ray imaging system comprises an X-ray imaging device for acquiring an X-ray image of the patient and an image processing apparatus according to the first aspect and any associated example.

According to a third aspect of the present invention, there is provided an image processing method according to claim 9.

According to an embodiment of the present invention, the method further comprises the step of detecting a presence of one or more pathologies in the second X-ray image.
According to an embodiment of the present invention, the method further comprises the step of providing a probability score of the one or more detected pathologies in the second X-ray image.

The image processing method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

According to a fourth aspect of the present invention, there is provided a computer program product comprising instructions which, when the program is executed by one or more processors, cause the one or more processors to carry out the steps of the method of the third aspect and any associated example.

According to a further aspect of the present invention, there is provided a computer-readable data carrier having stored thereon the computer program product.

It should be noted that the above embodiments may be combined with each other irrespective of the aspect involved. Accordingly, the method may be combined with structural features and, likewise, the apparatus and the system may be combined with features described above regarding the method.

These and other aspects of the present invention will become apparent from and elucidated with reference to the embodiments described hereinafter.

### BRIEF DESCRIPTION OF THE DRAWINGS

Exemplary embodiments of the invention will be described in the following with reference to the following drawings.
Fig 1 shows a flow chart describing an exemplary image processing method.
Fig. 2 schematically shows exemplary PA and LAT chest X-ray images.
Fig. 3 shows an example of a machine-learning model.
Fig. 4 shows a further example of a machine-learning model.
Fig. 5 shows a block diagram of an exemplary image processing apparatus.
Fig. 6 shows an example of a medical imaging system.

The figures are merely schematic representations and serve only to illustrate embodiments of the invention. Identical or equivalent elements are in principle provided with the same reference signs.

### DETAILED DESCRIPTION OF EMBODIMENTS

In the following, the approach is described in relation with chest X-ray images for the purposes of illustration. However, anyone of ordinary skill in the art will appreciate that the method and apparatus described above and below can be adapted to other body part including, but not limited to, joints, neck, spine, limbs, and other parts of the body. Accordingly, the following described examples are set forth without any loss of generality to, and without imposing limitations upon, the claimed invention.

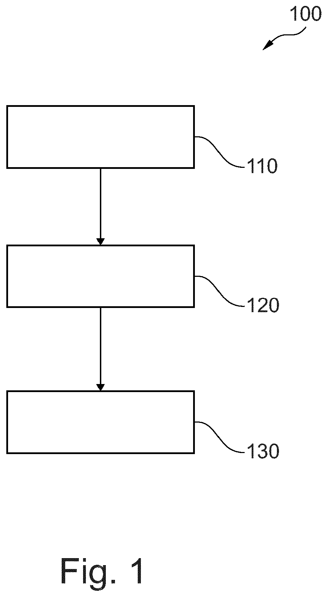

Fig. 1 illustrates a flow chart describing an exemplary image processing method 100. The image processing method 100 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 100 may be written in any combination of one or more programming languages, including an object-oriented programming languages and conventional procedural programming languages, such as the JAVA, SMALLTALK, C++, Python, or "C" programming languages or similar programming languages. For example, the exemplary image processing method may be implemented in an image processing apparatus 10 shown in Fig. 5.

At block 110, a first X-ray image having a first view of a body part of a patient is received. The first X-ray image may be received from an X-ray imaging apparatus or from a database, such as PACS (Picture Archiving and Communication System). Examples of the body part of the patient may include, but are not limited to, joints, neck, spine, limbs, or other parts of the body. For the purposes of illustration, the following approach is described in relation to the chest of the patient.

For chest X-ray images, the first view of the chest may be selected from a PA view, an AP view, a LAT view, a decubitus view, a lordotic view, an expiratory view, an inspiratory view, and an oblique view. The first X-ray image may be a two-dimensional image comprising image pixel data.

At block 120, based on the received first X-ray image, a second X-ray image having a second view of the body part of the patient is generated using a trained machine-learning model. The second view is different from the first view.

In one example, if the first X-ray image has a PA view of the chest, the second X-ray image may have e.g., a LAT view, a decubitus view, a lordotic view, an expiratory view, or an oblique view. For example, Fig. 2 schematically shows exemplary PA and LAT chest X-ray images.

In another example, if the first X-ray image has a LAT view, the second X-ray image may have an AP view, a PA view, a decubitus view, a lordotic view, an expiratory view, or an oblique view.

The machine-learning model may be a machine learning generative model for reproducing a relation between the first X-ray image having a first view of the body part and the second X-ray image having a second view of the body part. The machine learning generative model may be used to e.g., generate additional views in form of a synthetic X-ray image from the first X-ray image (e.g. PA data). In one example, the machine learning generative model may include a generative model of image synthesis using a probabilistic framework. In another example, the machine learning generative model may include a deep encoder-decoder network architecture, which is able to synthesize an X-ray image having a view of the body part into an X-ray image having a different view of the body part.

Fig. 3 shows an example of a machine-learning model that has an encoder-decoder network architecture. The architecture relies on finding a mapping between an input image and a desired image. The input image is an acquired X-ray image having a first view of the body part and the desired image is a synthetic X-ray image having a second view of the body part. In the example of Fig. 3, the exemplary encoder decoder network architecture is used for performing an image-to-image mapping for PA and LAT chest X-ray images.

The goal of the encoder decoder network architecture is to find the relationship, which associates the representations of X-ray images having different views of the same body part. In particular, the input layer L1 receives the first X-ray image having a first view of the body part, such as an X-ray image having a PA view of the chest shown in Fig. 2. Then, layers L2 and L3 aim to encode the input. The subsequent layers L4 and L5 essentially decode the information coming from the previous layers, providing at the output the desired second X-ray image having a second view of the body part, such as X-ray image having a LAT view of the chest shown in Fig. 2. The weights per-layer may be pre-trained based on a training dataset comprising a plurality of pairs of acquired X-ray images having the first and second views of the body part. The pre-training may rely on unsupervised learning.

In the inference phase, an X-ray image having the first view of the body part is provided to the layer L1 and into the entire network. The activations on the layer L5 are the output of the network and represent the actual synthetic X-ray image having a second view of the body part.

Optionally, as shown in Fig. 3, in additional to the X-ray image, additional data may be included in the training and inference process, e.g., by concatenating this information with the output of convolutional channels in the encoder or decoder channels. The additional data may comprise non-image patient data including, but not limited to, age of the patient, gender of the patient, presence of an implant, medication record associated with the patient, or any combination thereof. Additionally or alternatively, the additional data may comprise system data of the X-ray imaging system for acquiring the first X-ray image, which may include, but not limited to, view position, and peak kilovoltage (kVp).

Fig. 4 shows a further example of a machine-learning model with an additional discriminator, which is used to ensure the generation of realistically synthetic X-ray images.

In other words, the machine-learning model shown in Fig. 4 comprises a generator component and a discriminator component. The generator component comprises an encoder-decoder architecture configured to map the first X-ray image to the second X-ray image. An exemplary encoder-decoder architecture is shown in Fig. 3. The discriminator component comprises a discriminator that has been trained with concatenated image pairs to discriminate both. Each concatenated image pair comprises a first X-ray image pair comprising first and second X-ray images acquired by an X-ray imaging device, and a second X-ray image pair comprising the first X-ray image acquired by the X-ray imaging device and a second X-ray image generated by the image processing apparatus.

The discriminator may be pre-trained with concatenated image pairs, such as (real PA, real LAT) and (real PA, synthetic LAT), trying to discriminate both, and deriving the generation of more realistic synthetic X-ray images having a different view. It should be noted that the real PA and real LAT represent acquired X-ray images having a PA view and a LAT view, whereas the synthetic LAT represents an X-ray image having a LAT view generated by the image processing apparatus.

Similarly, additional data, such as non-image patient data and system data, may be included into the training and prediction process, e.g., by concatenating this information with the output of convolutional channels in the encoder, decoder, or discriminator.

Turning to Fig. 1, at block 130, the generated second X-ray image is provided e.g., to an electronic display, such as a display on the system console, at a PACS/diagnostic workstation. Alternatively or additionally, the generated second X-ray image may be provided to a storage medium.

In some examples, a presence of one or more pathologies may be detected in the second X-ray image, i.e., the synthetic X-ray image having a different view. For example, an AI-model, such as deep Convolution Neural Network (CNN) architectures, trained on large population data may be used to detect subtle signs of specific pathologies on the synthetic X-ray image. The AI-based detection may be done on the synthetic virtual projections. The original projection in the first X-Ray image may be used as additional input as well.

There is a dedicated selection of pathologies, which are typically diagnoses on projection images different from that at hand, e.g., on a lateral projection. The AI-model provides a probability score of one or multiple of such pathologies in the first X-ray image. A high probability score is used as a recommendation system to create another project image, i.e., the second image, from the same patient to assess the pathologies.

Fig. 5 illustrates an exemplary image processing apparatus 10. The image processing apparatus 10 comprises an input 12, one or more processors 14, and an output 16.

In general, the image processing apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 5 may show a limited number of components by way of example, it can be appreciated that a greater or a fewer number of components may be employed for a given implementation.

In some implementations, the image processing apparatus 10 may be embodied as, or in, a device or apparatus, such as a server, workstation, or mobile device. The image processing apparatus 10 may comprise one or more microprocessors or computers, which execute appropriate software. The processor 14 of the image processing apparatus 10 may be embodied by one or more of these microprocessors or computers. The software may have been downloaded and/or stored in a corresponding memory, e.g., a volatile memory such as RAM or a non-volatile memory such as flash. The software may comprise instructions configuring the one or more processors to perform the functions described herein.

It is noted that the image processing apparatus 10 may be implemented with or without employing a processor, and also may be implemented as a combination of dedicated hardware to perform some functions and a processor (e.g., one or more programmed microprocessors and associated circuitry) to perform other functions. For example, the functional units of the image processing apparatus 10, e.g., the input 12, the one or more processors 14, and the output 16 may be implemented in the device or apparatus in the form of programmable logic, e.g., as a Field-Programmable Gate Array (FPGA). In general, each functional unit of the apparatus may be implemented in the form of a circuit.

In some implementations, the image processing apparatus 10 may also be implemented in a distributed manner. For example, some or all units of the image processing apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

The processor(s) 14 may execute instructions to perform the image processing method described herein, which is explained in detail with respect to the embodiment shown in Fig. 1.

The input 12 and the output 14 may include hardware and/or software to enable the image processing apparatus 10 to receive a data input, and to communicate with other devices and/or a network. The input 12 may receive the data input via a wired connection or via a wireless connection. The output 16 may also provide cellular telephone communications, and/or other data communications for the image processing apparatus 10.

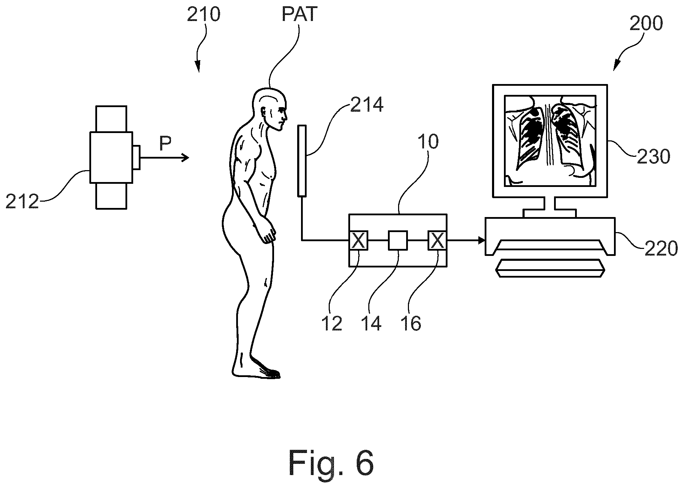

Fig. 6 shows schematically and exemplary an example of an X-ray imaging system 200. In this example, the exemplary X-ray imaging system is a chest radiography imaging system 200. The chest radiography imaging system 200 comprising an image processing apparatus 10, an X-ray imaging device 210, and a system console 220.

The X-ray imaging system 210 comprises an X-ray source 212 and an X-ray detector 214. The X-ray detector 214 is spaced from the X-ray source 212 to accommodate a patient PAT to be imaged.

In general, during an image acquisition, a collimated X-ray beam (indicated with arrow P) emits from the X-ray source 212, passes through the patient PAT at a region of interest (ROI), experiences attenuation by interaction with matter therein, and the attenuated beam then strikes the surface of the X-ray detector 214. The density of the organic material making up the ROI determines the level of attenuation. That is the rib cage and lung tissue in the chest radiography imaging examination. High-density material (such as bone) causes higher attenuation than less dense materials (such as lung tissue). The registered digital values for the X-ray are then consolidated into an array of digital values forming an X-ray projection image for a given acquisition time and projection direction.

Overall operation of the X-ray imaging system 210 may be controlled by an operator from the system console 220. The system console 220 may be coupled to a screen or monitor 230 on which the acquired X-ray images or imager settings may be viewed or reviewed. An operator such as a medical lab technical can control via the system console 220 an image acquisition run by releasing individual X-ray exposures for example by actuating a joystick or pedal or other suitable input means coupled to the system console 220.

In the example of Fig. 6, the patient PAT stands facing a flat surface behind which is the X-ray detector 214. According to a different example (not shown), the X-ray imaging system 210 is of the C-arm type, and the patient PAT is actually lying on an examination table instead of standing.

As described above, the image processing apparatus 10 may be any computing device, including desktop and laptop computers, smartphones, tablets, etc. The image processing apparatus 10 may be a general-purpose device or a device with a dedicated unit of equipment suitable for providing the functionality as described herein. In the example of Fig. 6, the components of the image processing apparatus 10 are shown as integrated in one single unit. However, in alternative examples, some or all components may be arranged as separate modules in a distributed architecture and connected in a suitable communication network. The image processing apparatus 10 and its components may be arranged as dedicated FPGAs or as hardwired standalone chips, such as the image processing apparatus shown in Fig. 6. In some examples (not shown), the image processing apparatus 10 or some of its components may be resident in the console 230 running as software routines.

In operation, an X-ray image acquired by the X-ray imaging device 210 is provided to the image processing apparatus 10. The acquired X-ray image may have a PA view of the chest of the patient PAT. The image processing apparatus 10 may generate an additional view (e.g., LAT image) from the PA data. The result may be displayed directly on the system console 220 or at a PACS/diagnostic workstation (not shown) to allow a radiologist to assess pathologies in a different view.

In another exemplary embodiment of the present invention, a computer program or a computer program element is provided that is characterized by being adapted to execute the method steps of the method according to one of the preceding embodiments, on an appropriate system.

The computer program element might therefore be stored on a computer, which might also be part of an embodiment of the present invention. This computer may be adapted to perform or induce a performing of the steps of the method described above. Moreover, it may be adapted to operate the components of the above-described apparatus. The computer can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it wherein the computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. Measures re-cited in mutually different dependent claims may be advantageously combined. Any reference signs in the claims should not be construed as limiting the scope.

## Claims

1. An image processing apparatus (10), comprising:
an input (12) configured to receive a first X-ray image obtained in an image acquisition, wherein the first X-ray image has a first view of a body part of a patient (PAT);
a processor (14) configured to:
detect a presence of one or more pathologies in the first X-ray image;
provide a probability score of the one or more detected pathologies in the first X-ray image;
determine, based on the probability score, whether to generate, based on the received first X-ray image, a second X-ray image having a second view of the body part of the patient (PAT) using a pre-trained machine-learning model to further assess the one or more detected pathologies, wherein the second view is different from the first view; and
an output (16) configured to provide the generated second X-ray image.

2. The image processing apparatus (10) according to claim 1,
wherein the input (12) is further configured to receive non-image patient data of the patient (PAT); and
wherein the pre-trained machine-learning model is further configured to apply the received non-image patient data to generate the second X-ray image.

3. The image processing apparatus (10) according to claim 1 or 2,
wherein the input (10) is further configured to receive system data of an X-ray imaging apparatus for acquiring the first X-ray image of the patient (PAT); and
wherein the pre-trained machine-learning model is further configured to apply the received system data to generate the second X-ray image.

4. The image processing apparatus (10) according to any one of claims 1 to 3,
wherein the pre-trained machine-learning model comprises an encoder-decoder architecture.

5. The image processing apparatus (10) according to claim 4,
wherein the pre-trained machine-learning model comprises a generator component and a discriminator component;
wherein the generator component comprises the encoder-decoder architecture configured to map the first X-ray image to the second X-ray image; and
wherein the discriminator component comprises a discriminator that has been trained with concatenated image pairs to discriminate both, wherein each concatenated image pair comprises a first X-ray image pair comprising first and second X-ray images acquired by an X-ray imaging device and a second X-ray image pair comprising the first X-ray image acquired by the X-ray imaging device and a second X-ray image generated by the image processing apparatus.

6. The image processing apparatus (10) according to any one of the preceding claims,
wherein the processor (12) is further configured to detect a presence of one or more pathologies in the second X-ray image.

7. The image processing apparatus (10) according to claim 6,
wherein the processor (12) is further configured to provide a probability score of the one or more detected pathologies in the second X-ray image.

8. An X-ray imaging system (200), the system comprising:
- an X-ray imaging device (210) for acquiring an X-ray image of a patient (PAT); and
- an image processing apparatus (10) according to any one of the preceding claims.

9. An image processing method (100), the method comprising:
- receiving (110) a first X-ray image obtained in an image acquisition, wherein the first X-ray image has a first view of a body part of a patient (PAT);
- detecting a presence of one or more pathologies in the first X-ray image,
- providing a probability score of the one or more detected pathologies in the first X-ray image; and
- determining, based on the probability score, whether to generate (120), based on the received first X-ray image, a second X-ray image having a second view of the body part of the patient (PAT) using a pre-trained machine-learning model to further assess the one or more detected pathologies, wherein the second view is different from the first view; and
- providing (130) the generated second X-ray image.

10. The image processing method according to claim 9, further comprising:
- receiving non-image patient data of the patient (PAT); and
wherein the pre-trained machine-learning model is further configured to apply the received non-image patient data to generate the second X-ray image.

11. The image processing method according to claim 9 or 10, further comprising:
- receiving system data of an X-ray imaging apparatus for acquiring the first X-ray image of the patient (PAT); and
wherein the pre-trained machine-learning model is further configured to apply the received system data to generate the second X-ray image.

12. The image processing method according to any of claims 9 to 11, further comprising:
- detecting a presence of one or more pathologies in the second X-ray image.

13. The image processing method according to claim 12, further comprising:
- providing a probability score of the one or more detected pathologies in the second X-ray image.

14. A computer program product comprising instructions which, when executed by one or more processors, cause the one or more processors to carry out the steps of the method of any one of claims 9 to 13.

15. A computer-readable data carrier having stored thereon the computer program product of claim 14.

## Patentansprüche

1. Bildverarbeitungseinrichtung (10), umfassend:
eine Eingabe (12), die dafür konfiguriert ist, ein erstes Röntgenbild zu empfangen, das in einer Bildaufnahme erlangt wurde, wobei das erste Röntgenbild eine erste Ansicht eines Körperteils eines Patienten (PAT) aufweist;
einen Prozessor (14), der dafür konfiguriert ist:
ein Vorliegen einer oder mehrerer Pathologien im ersten Röntgenbild zu erkennen;
eine Wahrscheinlichkeitspunktzahl der einen oder mehreren im ersten Röntgenbild erkannten Pathologien bereitzustellen;
auf der Grundlage der Wahrscheinlichkeitspunktzahl zu bestimmen, ob auf der Grundlage des empfangenen ersten Röntgenbildes ein zweites Röntgenbild, das eine zweite Ansicht des Körperteils des Patienten (PAT) aufweist, unter Verwendung eines vortrainierten Maschinenlernmodells erzeugt werden soll, um die eine oder mehreren erkannten Pathologien weiter zu beurteilen, wobei die zweite Ansicht sich von der ersten Ansicht unterscheidet; und
eine Ausgabe (16), die dafür konfiguriert ist, das erzeugte zweite Röntgenbild bereitzustellen.

2. Bildverarbeitungseinrichtung (10) nach Anspruch 1,
wobei die Eingabe (12) weiter dafür konfiguriert ist, Nicht-Bild-Patientendaten des Patienten (PAT) zu empfangen; und
wobei das vortrainierte Maschinenlernmodell weiter dafür konfiguriert ist, die empfangenen Nicht-Bild-Patientendaten anzuwenden, um das zweite Röntgenbild zu erzeugen.

3. Bildverarbeitungseinrichtung (10) nach Anspruch 1 oder 2,
wobei die Eingabe (10) weiter dafür konfiguriert ist, Systemdaten einer Röntgen-Bildgebungseinrichtung zum Aufnehmen des ersten Röntgenbildes des Patienten (PAT) zu empfangen; und
wobei das vortrainierte Maschinenlernmodell weiter dafür konfiguriert ist, die empfangenen Systemdaten anzuwenden, um das zweite Röntgenbild zu erzeugen.

4. Bildverarbeitungseinrichtung (10) nach einem der Ansprüche 1 bis 3,
wobei das vortrainierte Maschinenlernmodell eine Codierer-Decodierer-Architektur umfasst.

5. Bildverarbeitungseinrichtung (10) nach Anspruch 4,
wobei das vortrainierte Maschinenlernmodell eine Generatorkomponente und eine Diskriminatorkomponente umfasst;
wobei die Generatorkomponente die Codierer-Decodierer-Architektur umfasst, die dafür konfiguriert ist, das erste Röntgenbild auf das zweite Röntgenbild abzubilden; und
wobei die Diskriminatorkomponente einen Diskriminator umfasst, der mit verketteten Bildpaaren trainiert worden ist, um zwischen beiden zu unterscheiden, wobei jedes verkettete Bildpaar umfasst: ein erstes Röntgenbildpaar, das ein erstes und ein zweites Röntgenbild umfasst, die durch eine Röntgen-Bildgebungsvorrichtung aufgenommen wurden, und ein zweites Röntgenbildpaar, welches das durch die Röntgen-Bildgebungsvorrichtung aufgenommene erste Röntgenbild und ein durch die Bildverarbeitungseinrichtung erzeugtes zweites Röntgenbild umfasst.

6. Bildverarbeitungseinrichtung (10) nach einem der vorstehenden Ansprüche,
wobei der Prozessor (12) weiter dafür konfiguriert ist, ein Vorliegen der einen oder mehreren Pathologien im zweiten Röntgenbild zu erkennen.

7. Bildverarbeitungseinrichtung (10) nach Anspruch 6,
wobei der Prozessor (12) weiter dafür konfiguriert ist, einen Wahrscheinlichkeitspunktzahl der einen oder mehreren im zweiten Röntgenbild erkannten Pathologien bereitzustellen.

8. Röntgenbildgebungssystem (200), wobei das System umfasst:
- eine Röntgen-Bildgebungsvorrichtung (210) zum Aufnehmen eines Röntgenbildes eines Patienten (PAT); und
- eine Bildverarbeitungseinrichtung (10) nach einem der vorstehenden Ansprüche,

9. Bildverarbeitungsverfahren (100), wobei das Verfahren umfasst:
- Empfangen (110) eines ersten Röntgenbildes, das in einer Bildaufnahme erlangt wurde, wobei das erste Röntgenbild eine erste Ansicht eines Körperteils eines Patienten (PAT) aufweist;
- Erkennen eines Vorliegens einer oder mehrerer Pathologien im ersten Röntgenbild,
- Bereitstellen eines Wahrscheinlichkeitspunktzahls der einen oder mehreren im ersten Röntgenbild erkannten Pathologien; und
- Bestimmen auf der Grundlage des Wahrscheinlichkeitspunktzahls, ob auf der Grundlage des empfangenen ersten Röntgenbildes ein zweites Röntgenbild, das eine zweite Ansicht des Körperteils des Patienten (PAT) aufweist, unter Verwendung eines vortrainierten Maschinenlernmodells erzeugt werden soll (120), um die eine oder mehreren erkannten Pathologien weiter zu beurteilen, wobei die zweite Ansicht sich von der ersten Ansicht unterscheidet; und
- Bereitstellen (130) des erzeugten zweiten Röntgenbildes.

10. Bildverarbeitungsverfahren nach Anspruch 9, weiter umfassend:
- Empfangen von Nicht-Bild-Patientendaten des Patienten (PAT); und
wobei das vortrainierte Maschinenlernmodell weiter dafür konfiguriert ist, die empfangenen Nicht-Bild-Patientendaten anzuwenden, um das zweite Röntgenbild zu erzeugen.

11. Bildverarbeitungsverfahren nach Anspruch 9 oder 10, weiter umfassend:
- Empfangen von Systemdaten einer Röntgen-Bildgebungseinrichtung zum Aufnehmen des ersten Röntgenbildes des Patienten (PAT); und
wobei das vortrainierte Maschinenlernmodell weiter dafür konfiguriert ist, die empfangenen Systemdaten anzuwenden, um das zweite Röntgenbild zu erzeugen.

12. Bildverarbeitungsverfahren nach einem der Ansprüche 9 bis 11, weiter umfassend:
- Erkennen eines Vorliegens einer oder mehrerer Pathologien im zweiten Röntgenbild.

13. Bildverarbeitungsverfahren nach Anspruch 12, weiter umfassend:
- Bereitstellen eines Wahrscheinlichkeitspunktzahls der einen oder mehreren im zweiten Röntgenbild erkannten Pathologien.

14. Computerprogrammprodukt, das Anweisungen umfasst, die, wenn sie von einem oder mehreren Prozessoren ausgeführt werden, den einen oder mehrere Prozessoren veranlassen, die Schritte des Verfahrens nach einem der Ansprüche 9 bis 13 auszuführen.

15. Computerlesbarer Datenträger, auf dem das Computerprogrammprodukt nach Anspruch 14 gespeichert ist.

## Revendications

1. Appareil de traitement d'image (10), comprenant :
une entrée (12) configurée pour recevoir une première image radiographique obtenue lors d'une acquisition d'image, dans lequel la première image radiographique présente une première vue d'une partie du corps d'un patient (PAT) ;
un processeur (14) configuré pour :
détecter la présence d'une ou plusieurs pathologies sur la première image radiographique ;
fournir un score de probabilité de la ou des pathologies détectées sur la première image radiographique ;
déterminer, en fonction du score de probabilité, s'il convient de générer, à partir de la première image radiographique reçue, une seconde image radiographique présentant une seconde vue de la partie du corps du patient (PAT) à l'aide d'un modèle d'apprentissage automatique pré-entraîné afin d'évaluer plus précisément la ou les pathologies détectées, dans lequel la seconde vue est différente de la première ;
une sortie (16) configurée pour fournir la seconde image radiographique générée.

2. Appareil de traitement d'image (10) selon la revendication 1,
dans lequel l'entrée (12) est en outre configurée pour recevoir des données patient non-imagées du patient (PAT) ; et
dans lequel le modèle d'apprentissage automatique pré-entraîné est configuré en outre pour appliquer les données patient non-imagées reçues afin de générer la seconde image radiographique.

3. Appareil de traitement d'image (10) selon la revendication 1 ou 2,
dans lequel l'entrée (10) est en outre configurée pour recevoir les données système d'un appareil d'imagerie à rayons X permettant d'acquérir la première image radiographique du patient (PAT) ; et
dans lequel le modèle d'apprentissage automatique pré-entraîné est configuré en outre pour appliquer les données système reçues afin de générer la seconde image radiographique.

4. Appareil de traitement d'image (10) selon l'une quelconque des revendications 1 à 3,
dans lequel le modèle d'apprentissage automatique pré-entraîné comprend une architecture encodeur-décodeur.

5. Appareil de traitement d'image (10) selon la revendication 4,
dans lequel le modèle d'apprentissage automatique pré-entraîné comprend un composant générateur et un composant discriminateur ;
dans lequel le composant générateur comprend l'architecture encodeur-décodeur configurée pour faire correspondre la première image radiographique à la seconde image radiographique ; et
dans lequel le composant discriminateur comprend un discriminateur qui a été entraîné avec des paires d'images concaténées pour discriminer les deux, dans lequel chaque paire d'images concaténées comprend une première paire d'images radiographiques comprenant une première et une seconde image radiographique acquise par un dispositif d'imagerie radiographique et une seconde paire d'images radiographiques comprenant la première image radiographique acquise par le dispositif d'imagerie radiographique et une seconde image radiographique générée par l'appareil de traitement d'image.

6. Appareil de traitement d'image (10) selon l'une quelconque des revendications précédentes,
dans lequel le processeur (12) est en outre configuré pour détecter la présence d'une ou plusieurs pathologies dans la seconde image radiographique.

7. Appareil de traitement d'image (10) selon la revendication 6,
dans lequel le processeur (12) est en outre configuré pour fournir un score de probabilité de la ou des pathologies détectées dans la seconde image radiographique.

8. Système d'imagerie à rayons X (200), le système comprenant :
- un dispositif d'imagerie à rayons X (210) pour l'acquisition d'une image radiographique d'un patient (PAT) ; et
- un appareil de traitement d'image (10) selon l'une quelconque des revendications précédentes.

9. Procédé de traitement d'image (100), le procédé comprenant :
- la réception (110) d'une première image radiographique obtenue lors d'une acquisition d'image, dans laquelle la première image radiographique présente une première vue d'une partie du corps d'un patient (PAT) ;
- détecter la présence d'une ou plusieurs pathologies sur la première image radiographique,
- la fourniture d'un score de probabilité pour une ou plusieurs pathologies détectées sur la première image radiographique ; et
- la détermination, en fonction du score de probabilité, s'il convient de générer (120), à partir de la première image radiographique reçue, une seconde image radiographique présentant une seconde vue de la partie du corps du patient (PAT) à l'aide d'un modèle d'apprentissage automatique pré-entraîné afin d'évaluer plus précisément la ou les pathologies détectées, dans lequel cette seconde vue est différente de la première ; et
- la fourniture (130) de la seconde image radiographique générée.

10. Procédé de traitement de données selon la revendication 9, comprenant en outre :
- la réception des données non-imagées du patient (PAT) ; et
dans lequel le modèle d'apprentissage automatique pré-entraîné est configuré en outre pour appliquer les données patient non-imagées reçues afin de générer la seconde image radiographique.

11. Procédé de traitement de données selon la revendication 9 ou 10, comprenant en outre :
- la réception de données du système d'un appareil d'imagerie radiographique pour l'acquisition de la première image radiographique du patient (PAT) ; et
dans lequel le modèle d'apprentissage automatique pré-entraîné est configuré en outre pour appliquer les données système reçues afin de générer la seconde image radiographique.

12. Procédé de formation d'image selon l'une quelconque des revendications 9 à 11, comprenant en outre :
- la détection de la présence d'une ou plusieurs pathologies sur la seconde image radiographique.

13. Procédé de traitement d'image biométrique selon la revendication 12, comprenant en outre :
- la fourniture d'un score de probabilité de la ou des pathologies détectées sur la seconde image radiographique.

14. Produit de programme informatique comprenant des instructions qui, lorsqu'elles sont exécutées par un ou plusieurs processeurs, amènent les un ou plusieurs processeurs à mettre en œuvre le procédé selon l'une quelconque des revendications 9 à 13.

15. Support de données lisible par ordinateur sur lequel est stocké le produit de programme informatique selon la revendication 14.
